# EUROPEAN PATENT APPLICATION

(11) **EP 3 620 124 A1**
(43) Date of publication of application: **11.03.2020**
(21) Application number: 19794403.6
(22) Date of filing: 10.07.2019
(51) Int. Cl.: A61B 17/34, A61B 17/3205, A61F 2/10, A61B 17/00

(54) **PUNCHING NEEDLE AND HANDPIECE FOR EXTRACTING HAIR**

(30) Priority: 10.07.2018 KR 20180079941
(71) Applicant: Park, Jae Hyun, Seoul 06544 (KR); Park, Choon Bae, Iksan-si, Jeollabuk-do 54646 (KR)
(72) Inventor: Park, Jae Hyun, Seoul 06544 (KR); Park, Choon Bae, Iksan-si, Jeollabuk-do 54646 (KR)
(74) Representative: Flügel Preissner Schober Seidel
(86) International application number: PCT/KR2019/008496
(87) International publication number: WO 2020/013603

(57) **Abstract**

According to one embodiment of the present invention, a punching needle for a handpiece used to extract follicle from patient's skin is provided. According to one embodiment of the present invention, an accommodation space, into which follicle of extraction target hair may be inserted, may be formed in the inside of a front portion of the punching needle, and a punching portion of contacting with the patient's skin and punching the patient's skin may be provided in the front end portion of the punching needle. According to one embodiment of the present invention, the punching portion may comprise a first punching portion and a second punching portion which have different-shaped cutting edges, and a first cutting edge provided in the first punching portion may be formed in a shape that is sharper than that of a second cutting edge provided in the second punching portion.

## Description

### [Technical Field]

The present invention relates to a punching needle and a handpiece which are used for extracting a hair from a patient's skin, and more particularly, to a punching needle and a handpiece which are capable of stably extracting hair while reducing a risk of damage to a follicle of extraction target hair or surrounding hair.

### [Background Art]

Hair transplantation is a procedure for transplanting new hairs to a site where hairs have been lost in the case of hair loss on human skin due to diseases or accidents. Hair transplantation may be classified into follicular unit strip surgery (FUSS) and follicular unit extraction (FUE) according to a method of obtaining a hair to be transplanted.

Follicular unit strip surgery refers to a procedure in which a certain scalp area is incised in a donor site, a hair follicle is then extracted from the incised scalp and transplanted to a necessary site, and the incision area of the donor site is stitched with a suture. Such follicular unit strip surgery is currently most used as a method for follicular unit transplantation surgery. However, this surgery has disadvantages in that, because the scalp in the donor site is incised, it causes great pain for a patient and a large surgical scar is inevitably generated at the incision area.

Follicular unit extraction is a procedure that has been introduced to overcome the disadvantages of the above-described follicular unit strip surgery. Follicular unit extraction refers to a procedure in which a hair to be transplanted is extracted from patient's skin using a hair follicle separator (i.e., handpiece) without incising a scalp of the patient. Follicular unit extraction has advantages in that, since this procedure is performed without incising the patient's skin, patient's fear on skin incision may be eliminated and formation of a large surgical scar over a wide area after the procedure may be prevented.

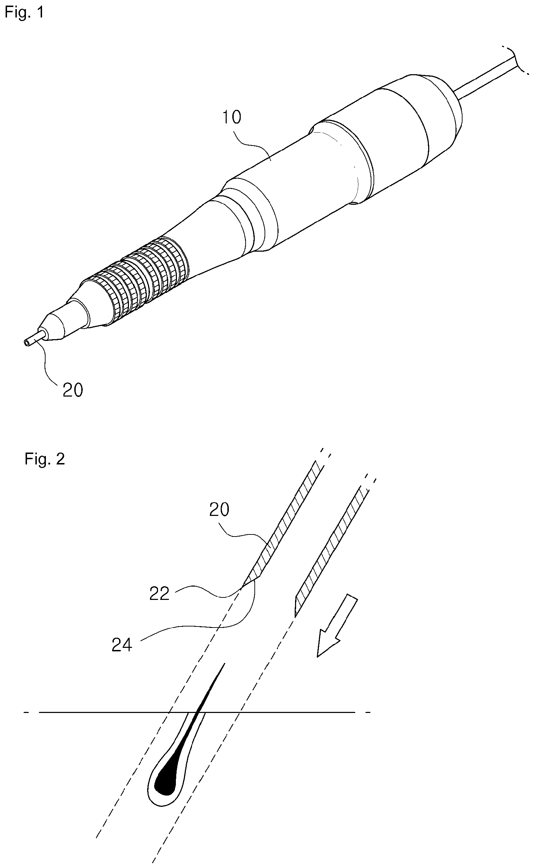

In FIG. 1, a handpiece 10 used for extracting a hair to be transplanted in follicular unit extraction is exemplarily illustrated. By inserting a punching needle 20 mounted in the front of the handpiece 10 into a patient's skin and puncturing the patient's skin, the handpiece 10 performs a function of separating a follicle of the extraction target hair from surrounding tissues (sebaceous glands, arrector pili muscles, and the like). Generally, the handpiece 10 includes an electric motor and a spindle assembly provided therein, and is configured to insert the punching needle 20 into the patient's skin while rotating the punching needle 20 with a rotational force of the electric motor. When the hair follicle is separated from the surrounding tissues using the handpiece 10, the hair may be extracted to the outside by pulling the hair with medical equipment such as forceps.

However, since a punching operation of extracting the hair using the handpiece is a blind procedure which should be performed in a state that the follicle located below a skin layer is not visually directly identified, the follicle may be easily cut or damaged by the punching needle during the follicle is separated from the surrounding tissues using the punching needle. Furthermore, in order to effectively separate the follicle from the surrounding tissues while easily puncturing the patient's skin, the punching needle 20 is usually formed in a structure that a sharp cutting edge 22 is provided at a front end portion. (For example, as shown in FIG. 2, the punching needle 20 is formed such that an inclined surface 24 is formed on an inner circumferential surface of the front end portion of the punching needle 20 and thus the sharp cutting edge 22 is formed on a distal end of the front end portion of the punching needle 20.) Consequently, the follicle of the extraction target hair or follicles located therearound may be easily damaged by the sharp cutting edge.

In order to resolve the above problem, various attempts for improving a structure of the punching needle are being carried in the field of hair transplantation. For example, referring to Patent Document 1, a technique for improving a shape of a distal end portion of a punching needle to reduce a risk of cutting or damaging a follicle of extraction target hair by a cutting edge of a punching needle is disclosed.

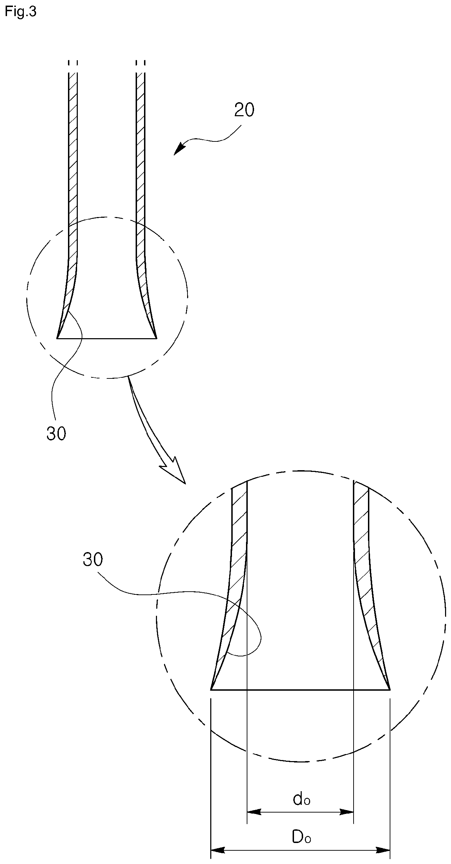

Specifically, a punching needle 20 disclosed in Patent Document 1 is formed in a structure that the front end portion of the punching needle 20, which is inserted into patient's skin, is formed so as to outwardly extend in a radial direction toward a distal end, thereby reducing the risk of cutting or damaging the follicle of the extraction target hair by the cutting edge which is provided in the front end portion of the punching needle, as shown in FIG. 3.

However, since an inner diameter and the outer diameter of the punching needle 20 having the above-described structure are formed to outwardly extend in a radial direction toward the distal end, a diameter Do of the cutting edge which punches the patient's skin is inevitably increased, and thus a risk of damaging surrounding follicles located adjacent to the follicle of the extraction target hair by the cutting edge is increased.

In addition, owing to the structure in which the distal end of the punching needle 20 expands, a large diameter difference between the outer diameter Do of the cutting edge and an inner diameter do of the punching needle is generated and an expanded surface 30 having a large area is formed on an end portion of the inner circumference of the punching needle. Accordingly, the patient's skin is pressurized by the expanded surface 30 and a position of the follicle located therein is unintendedly moved, such that the follicle of the extraction target hair or the surrounding follicles may be brought into contact with the cutting edge of the punching needle to be easily cut or damaged by the cutting edge.

### (Prior Art Document)

Patent Document 1: U.S. Patent No. 8,876,847

### [Technical Problem]

The present invention is directed to providing a punching needle and a handpiece which are capable of stably extracting hair while reducing a risk of damage to a follicle of extraction target hair or surrounding hair, for resolving the afore-mentioned technical problems of the hair extraction.

### [Technical Solution]

Technical features of the present invention for achieving the above-described objectives are as follows.

According to one embodiment of the present invention, a punching needle for a handpiece used to extract follicle from patient's skin is provided. According to one embodiment of the present invention, an accommodation space, into which follicle of extraction target hair may be inserted, may be formed in the inside of a front portion of the punching needle, and a punching portion of contacting with the patient's skin and punching the patient's skin may be provided in the front end portion of the punching needle. According to one embodiment of the present invention, the punching portion may comprise a first punching portion and a second punching portion which have different-shaped cutting edges, and a first cutting edge provided in the first punching portion may be formed in a shape that is sharper than that of a second cutting edge provided in the second punching portion.

According to one embodiment of the present invention, the first punching portion may be formed to decrease a thickness of the punching needle toward a front side such that the first cutting edge having a sharp tip may be formed at the front end portion.

According to one embodiment of the present invention, the first punching portion may have a wedge portion of a recessed shape on an outer circumference of the front portion such that the first cutting edge having the sharp tip may be formed at the front portion of the first punching portion due to the wedge portion, and the first cutting edge formed at the front portion of the first punching portion may be formed in a direction that is obliquely inclined with respect to a central axis of the punching needle due to the wedge portion.

According to one embodiment of the present invention, the wedge portion formed on the outer circumference of the front portion of the first punching portion may be formed of a recess of a triangular shape having a front inclined surface and a rear inclined surface.

According to one embodiment of the present invention, the first punching portion may comprise a dumbbell-shaped portion having a diameter reduction portion which is located at the front end portion and of which an outer diameter and an inner diameter become smaller toward a rear side; a diameter expansion portion which is located at the rear side of the diameter reduction portion and of which an outer diameter and an inner diameter becomes larger toward a rear side; and a connection portion which is formed in a cylindrical tube shape and which connects between the diameter reduction portion and the diameter expansion portion, and the first cutting edge having the sharp tip may be formed at the front end portion of the first punching portion.

According to one embodiment of the present invention, an end portion of the second cutting edge provided in the second punching portion may be rounded so that the portion, which comes into contact with the patient's skin, may be formed in a blunt shape.

According to one embodiment of the present invention, the first punching portion may be formed such that an entirety or a part of the front end portion of the first punching portion protrudes forward than the front end portion of the second punching portion.

According to one embodiment of the present invention, an entire or a portion of the second punching portion may be formed of a transparent material.

According to one embodiment of the present invention, the punching needle may further comprise a cutout portion formed to extend rearward in a length direction of the punching needle at one side of the front portion of the punching needle.

According to one embodiment of the present invention, a handpiece used to extract hair from patient's skin is provided. The handpiece according to one embodiment of the present invention may comprise a housing, a power generator provided in the housing, a spindle assembly configured to transmit a rotating force generated from the power generator, and a punching needle engaged with a front portion of the spindle assembly and inserted into the patient's skin while being rotated due to the rotating force generated from the power generator. According to one embodiment of the present invention, an accommodation space, into which follicle of extraction target hair may be inserted, may be formed in the inside of a front portion of the punching needle, and a punching portion of contacting with the patient's skin and punching the patient's skin may be provided in the front end portion of the punching needle. According to one embodiment of the present invention, the punching portion may comprise a first punching portion and a second punching portion which have different-shaped cutting edges, and a first cutting edge provided in the first punching portion may be formed in a shape that is sharper than that of a second cutting edge provided in the second punching portion.

According to one embodiment of the present invention, the first punching portion may be formed to decrease a thickness of the punching needle toward a front side such that the first cutting edge having a sharp tip may be formed at the front end portion.

According to one embodiment of the present invention, the first punching portion may have a wedge portion of a recessed shape on an outer circumference of the front portion such that the first cutting edge having the sharp tip may be formed at the front portion of the first punching portion due to the wedge portion, and the first cutting edge formed at the front portion of the first punching portion may be formed in a direction that is obliquely inclined with respect to a central axis of the punching needle due to the wedge portion.

According to one embodiment of the present invention, the wedge portion formed on the outer circumference of the front portion of the first punching portion may be formed of a recess of a triangular shape having a front inclined surface and a rear inclined surface.

According to one embodiment of the present invention, the first punching portion may comprise a dumbbell-shaped portion having a diameter reduction portion which is located at the front end portion and of which an outer diameter and an inner diameter become smaller toward a rear side; a diameter expansion portion which is located at the rear side of the diameter reduction portion and of which an outer diameter and an inner diameter becomes larger toward a rear side; and a connection portion which is formed in a cylindrical tube shape and which connects between the diameter reduction portion and the diameter expansion portion, and the first cutting edge having the sharp tip may be formed at the front end portion of the first punching portion.

According to one embodiment of the present invention, an end portion of the second cutting edge provided in the second punching portion may be rounded so that the portion, which comes into contact with the patient's skin, may be formed in a blunt shape.

According to one embodiment of the present invention, the first punching portion may be formed such that an entirety or a part of the front end portion of the first punching portion protrudes forward than the front end portion of the second punching portion.

According to one embodiment of the present invention, an entire or a portion of the second punching portion may be formed of a transparent material.

According to one embodiment of the present invention, the punching needle may further comprise a cutout portion formed to extend rearward in a length direction of the punching needle at one side of the front portion of the punching needle.

According to one embodiment of the present invention, the power generator may be driven to alternately rotate the punching needle in both directions.

According to one embodiment of the present invention, the power generator may be driven to rotate the punching needle in the both directions within an angle range in which the first punching portion is formed.

According to one embodiment of the present invention, the power generator may be controlled to start rotating the punching needle in response to a manipulation command of an operator through a manipulator and, when the manipulation command of the operator through the manipulator is released, the power generator may be controlled to terminate the rotation of the punching needle in a state of returning the punching needle to the position before the punching needle is operated.

In addition, the punching needle and the handpiece according to the present invention may further comprise other additional featrures without departing from the technical spirit of the present invention.

### [Advantageous Effects]

A punching needle according to one embodiment of the present invention is configured such that a plurality of cutting edges having different shapes (specifically, a first cutting edge of a sharper shape and a second cutting edge having a less sharp shape than the first cutting edge) are formed at a front portion of the punching needle. Accordingly, the punching needle may be operated such that the punching process is started by punching the patient's skin using the first cutting edge having a sharp shape and then the second cutting edge having a less sharp shape may be involved in a punching process after the punching operation is begun by the first cutting edge. As such, the punching needle according to one embodiment of the present invention is configured such that a large portion of the cutting edge is formed in a less sharp shape (preferably, in a blunt shape with no shape edge), unlike the conventional punching needle in which an entirety of a cutting edge located at a front portion thereof is sharply formed. Accordingly, the punching needle according to one embodiment of the present invention can reduce the risk of damage to follicle of extraction target hair or surrounding follicle by the sharp cutting edge of the punching needle, and can effectively perform the punching process of punching the patient's skin by starting to punch the patient's skin with the first cutting edge having a sharp shape, like the conventional punching needle.

### [Description of Drawings]

FIG. 1 illustrates an example of a conventional handpiece which may be employed in a follicular unit extraction.
FIG. 2 illustrates an example of a state in which a hair follicle is separated by the punching needle.
FIG. 3 illustrates an example of a conventional punching needle having an extension, of which an inner diameter and an outer diameter are expanded, in an end portion of the punching needle.
FIG. 4 illustrates an example of a configuration of a punching system according to one embodiment of the present invention.
FIGS. 5 to 7 illustrate examples of a configuration of a handpiece according to one embodiment of the present invention.
FIGS. 8 and 9 illustrate an example of a punching needle according to one embodiment of the present invention.
FIGS. 10 to 16 illustrate examples of a punching needle according to another embodiment of the present invention.
FIG. 17 illustrates an example of a state in which a punching operation is performed by the punching needle according to one embodiment of the present invention.

### [Description of Reference Numerals]

- 100:: Punching system
- 200:: Handpiece
- 210:: Housing
- 220:: Power generator (electric motor)
- 230:: Power transmitting part (spindle assembly)
- 240:: Mounting part (collet chuck)
- 250:: Encoder
- 260:: Front cover
- 300:: Main body
- 400:: Manipulator
- 500:: Punching needle
- 510:: Mounting portion (of punching needle)
- 520:: Punching portion
- 520a:: First punching portion
- 522a:: First cutting edge
- 520b:: Second punching portion
- 522b:: Second cutting edge
- 530:: Wedge portion
- 530a:: Front inclined surface
- 530b:: Rear inclined surface
- 540:: Dumbbell-shaped portion
- 540a:: Diameter reduction portion
- 540b:: Connection portion
- 540c:: Diameter expansion portion
- 550:: Cutout portion

### [Modes of the Disclosure]

Hereinafter, exemplarily embodiments of the present invention will be described in detail with reference to the accompanying drawings so that those skilled in the art to which the present invention pertains may easily carry out the present invention.

In order to clearly describe the present invention, a detailed description on parts which are not related to the present invention will be omitted, and the same components will be described by the same reference numerals throughout the specification. In addition, since a shape and size of each component shown in the drawings are arbitrarily shown for convenience of explanation, the present invention is not necessarily limited to the illustrated shape and size. That is, specific shapes, structures, and characteristics described in the specification may be modified and implemented from one embodiment to another embodiment without departing from the spirit and scope of the present invention, and it should be understood that a position or arrangement of individual component may be changed without departing from the spirit and scope of the present invention. Therefore, the following detailed description is not intended to be construed in a limiting sense, and the scope of the present invention should be construed as encompassing the scope of the appended claims and all equivalents thereof.

### Punching needle and handpiece according to one embodiment of the present invention

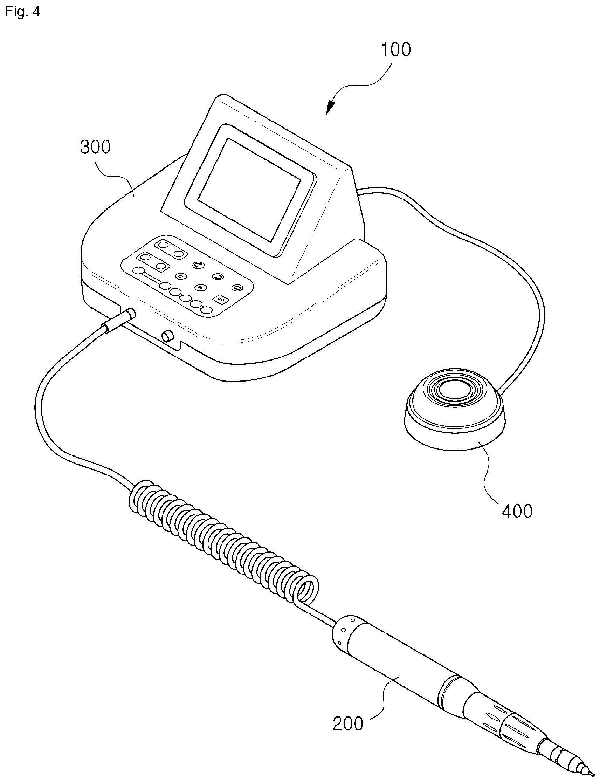

A punching system 100 according to one embodiment of the present invention is exemplarily illustrated in FIG. 4. As illustrated in FIG. 4, the punching system 100 may comprise a handpiece 200 configured to be grasped by an operator to perform a punching operation, a main body 300 configured to control operation of the handpiece 200, a manipulator 400 configured to turn on/off the operation of the handpiece 200. Although the manipulator 400 is implemented as a pedal in the embodiment shown in the drawing, the manipulator 400 may be provided in any form other than the pedal (e.g., an operation button provided on the handpiece 200 or the main body 300).

The handpiece 200 is configured such that a punching needle 500 is mounted at the front portion thereof to perform a function of puncturing a patient's skin with the punching needle 500 and separating a follicle of hair to be extracted from surrounding skin tissues. According to one embodiment of the present invention, the handpiece 200 may have components such as a power generator (for example, an electric motor), a power transmitting part (a spindle assembly), etc. therein, such as a conventional handpiece.

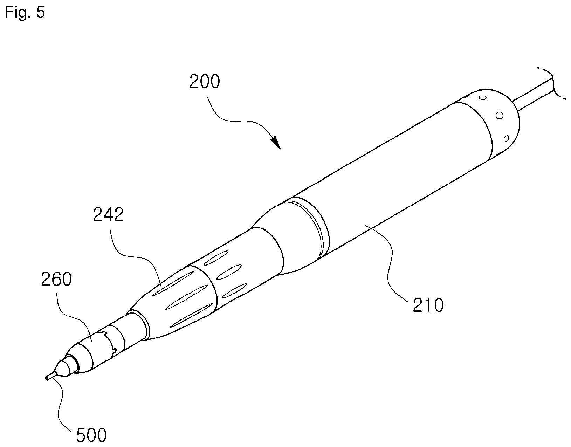

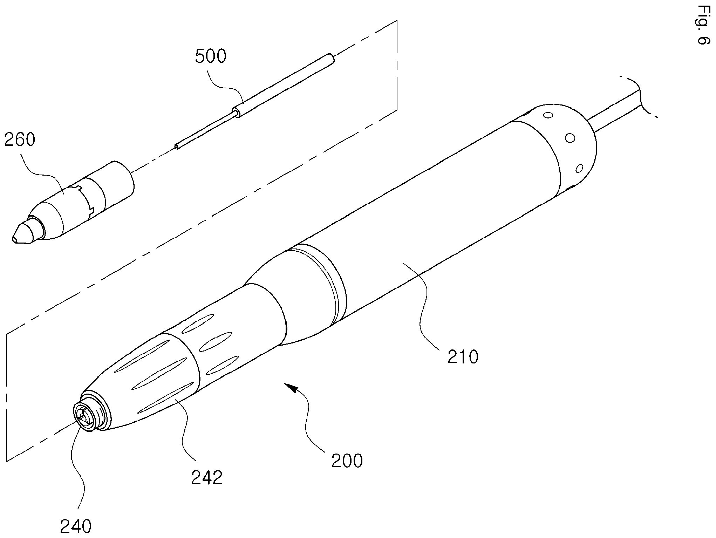

In FIGS. 5 to 7, the configuration of the handpiece 200 according to one embodiment of the present invention is exemplarily illustrated. As illustrated in the drawings, the handpiece 200 according to one embodiment of the present invention may comprise a housing 210 forming a body of the handpiece, a power generator 220 provided in the housing 210 to generate power required for rotating the punching needle 500, a power transmitting part 230 (spindle assembly) for transmitting the power generated by the power generator 220, and the like.

According to one embodiment of the present invention, a mounting part 240 with which the punching needle 500 is engaged may be provided at a front portion of the power transmitting part 230. The mounting part 240 may be implemented in a manner similar to that of a conventional handpiece. According to one embodiment of the present invention, the handpiece 200 may configured such that a collet chuck (the mounting part 240) is provided at the front portion of the power transmitting part 230, which is connected to the power generator 220, to hold the punching needle 500 through a chucking operation of the collet chuck. For example, when the operator rotates a collet chuck manipulating part 242 formed on the housing 210 of the handpiece 200 in one direction, a diameter of the collet chuck decreases and the collet chuck grasps the punching needle 500, and when the operator rotates the collet chuck manipulating part 242 in a direction opposite the one direction, an inner diameter of the collet chuck is increased and an engagement between the punching needle 500 and the collet chuck is released. The mounting part 240 may be formed in a structure and a shape which are similar to those of a conventional medical handpiece or an electric drill. The present invention is not characterized in a structure of the mounting part 240 on which the punching needle 500 is mounted, and thus a more detailed description thereof will be omitted herein.

The power generator 220 performs a function of generating a rotational force for rotating the punching needle 500 so as to allow the punching needle 500 to be easily inserted into the patient's skin. For example, the power generator 220 may be implemented using an electric motor or the like as in the conventional medical handpiece, and may be configured to rotate the punching needle 500 in one direction or both directions using a rotational force generated by the power generator 220. However, it may be more preferable that the power generator 220 of the handpiece 200 according to one embodiment of the present invention is configured to alternately rotate the punching needle 500 in the both directions so as to allow the punching needle 500 according to the embodiment of the present invention, which will be described below, to be effectively operated.

According to one embodiment of the present invention, an encoder 250 for detecting a rotation state of a motor may further be provided on the electric motor constituting the power generator 220 of the handpiece 200. For example, the encoder 250 may be mounted on a rear portion of the electric motor and may be configured to perform a function of detecting a rotation amount (rotation angle) of the electric motor and transmitting the detected rotation amount to a controller (not shown). Such a rotation state information of the electric motor, which is detected by the encoder 250, may be used to control rotation of the punching needle 500 or return the punching needle 500 to its original position before an operation after the punching operation is completed.

Meanwhile, a front cover 260 may be coupled to a front portion of the handpiece 200. The front cover 260 may be coupled to the front portion of the handpiece 200 to perform a function of adjusting a front exposed length of the punching needle 500.

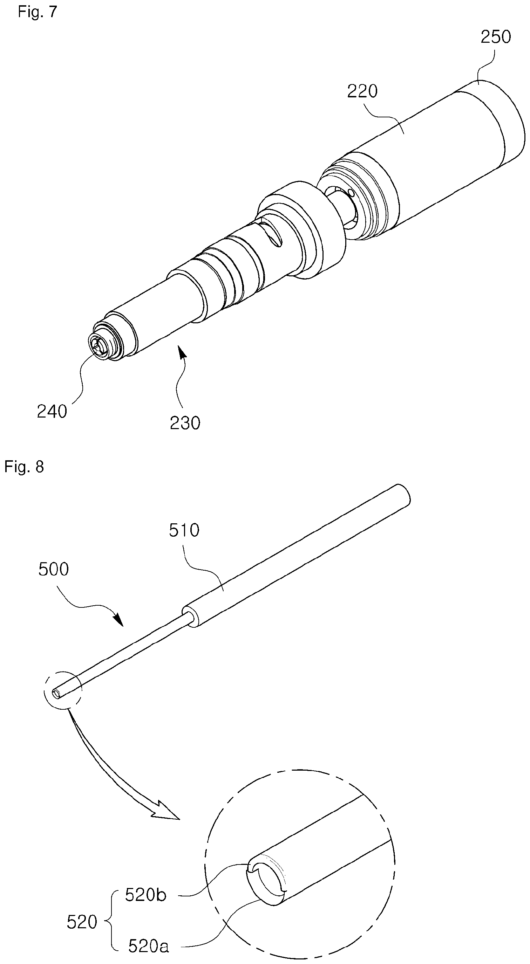

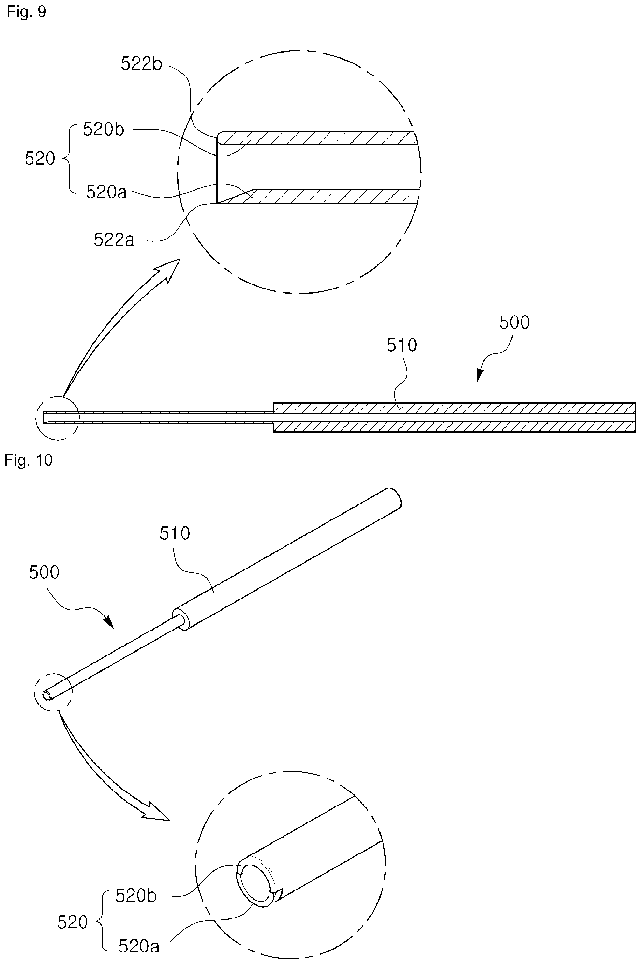

Next, the punching needle 500 which is engaged with the front portion of the handpiece 200 and inserted into the patient's skin will be described in detail. Referring to FIGS. 8 and 9, a configuration of the punching needle 500 according to one embodiment of the present invention is exemplarily illustrated.

According to one embodiment of the present invention, the punching needle 500 may be formed in a shape which is generally similar to that of a conventional medical punching needle. For example, the punching needle 500 may be formed in a hollow structure in which a central portion of the punching needle 500 is penetrated in a length direction and an accommodation space into which hair being extracted is inserted is formed at the inside of a front portion. Further, a mounting portion 510, which is used to mount the punching needle 500 on the handpiece 200, may be provided on an outer circumference of the punching needle 500. Since the punching needle 500 is generally formed to have a very small diameter, when the punching needle 500 is directly mounted on the handpiece 200, a mounting operation may be cumbersome and there is a probability that the punching needle 500 may be deformed in a process of mounting the punching needle 500 to the handpiece 200. Thus, it may be preferable to form the punching needle 500 such that the mounting portion 510 having a larger diameter is provided at a rear portion and the punching needle 500 is mounted on the handpiece 200 through the mounting portion 510. The mounting portion 510 may be integrally formed with the punching needle 500 or may be separately formed from the punching needle 500 and then coupled thereto. However, the mounting portion 510 is not necessarily provided, and the punching needle 500 may be formed to have a single diameter without the mounting portion 510 and mounted directly on the handpiece 200.

According to one embodiment of the present invention, the punching needle 500 may comprise a plurality of punching portions 520 having cutting edges of different shapes at a front end portion thereof. For example, the punching needle 500 according to one embodiment of the present invention may comprise a first punching portion 520a having a sharper cutting edge (a first cutting edge 522a) and a second punching portion 520b having a less sharp cutting edge (a second cutting edge 522b).

According to one embodiment of the present invention, the first punching portion 520a, which is formed at a portion of the front portion of the punching needle 500, may be formed in a structure in which a thickness of the punching needle is reduced toward the front side such that the first cutting edge 522a (sharp edge) having a sharp tip is formed at an end portion of the punching needle 500 in an axial direction, and the second punching portion 520b may be formed such that an end portion thereof is rounded and the second cutting edge 522b in contact with the patient's skin is formed in a blunt shape (which is a shape having no sharp edge) (a blunt punch or a dull punch).

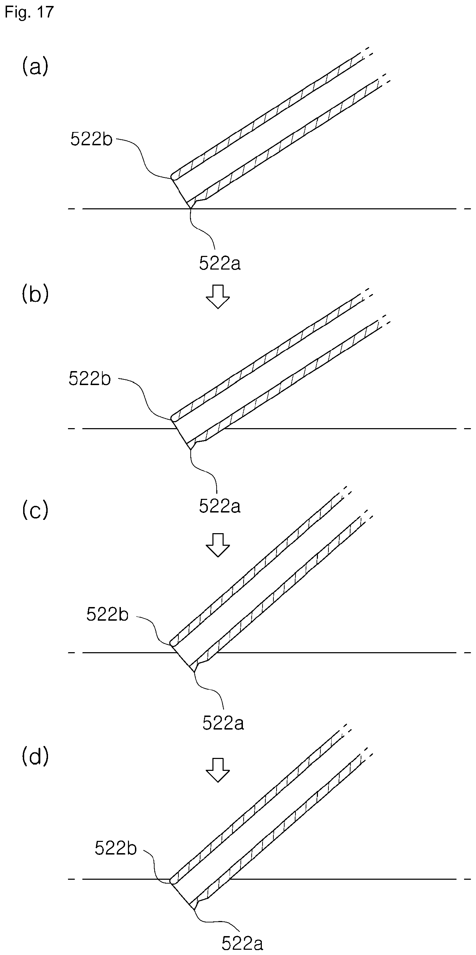

Generally, as shown in FIGS. 2 and 17, when a punching operation for extracting a hair from the patient's skin using the handpiece 200 is performed, the punching needle 500 is inserted into the patient's skin in a state of being obliquely inclined with respect to the patient's skin. Accordingly, at the beginning of the punching operation, an entirety of the front end portion of the punching needle 500 does not come into contact with the patient's skin, but only a portion of the front end portion of the punching needle 500 starts to punch the patient's skin in a state of coming into contact with the patient's skin. (For example, in the states shown in FIGS. 2 and 17, only end portion of the punching needles located at lower portions of the drawings comes into contact with the patient's skin to perform the punching operation.)

In consideration of the above, in the punching needle 500 according to one embodiment of the present invention, the first cutting edge 522a is formed as a cutting edge having a sharp tip shape so as to come into contact with the patient's skin to start to effectively punch the patient's skin, and the second cutting edge 522b which subsequently performs the punching operation is formed as a shape that is less sharper than that of the first cutting edge 522b (e.g., as shown in the drawings, a cutting edge of a blunt shape having a rounded end portion). With the above-described structure, at the beginning of the punching operation, the first cutting edge 522a having a sharp tip starts to punch an epidermal layer of the skin that is a relatively hard, and the second cutting edge 522b is involved in the punching operation after the punching operation is begun by the first cutting edge 522a. As such, since the second cutting edge 522b is involved in the punching operation in a state in which the patient's skin is punched to some extent by the first cutting edge 522a, even when the second cutting edge 522b is not formed to be relatively sharp, the punching operation may be smoothly performed. Further, unlike the conventional punching needle (e.g., the punching needle illustrated in FIGS. 2 and 3), since a portion of the cutting edge which is formed on the punching needle (e.g., the portion that the second cutting edge 522b is formed), is formed in an unsharp shape in the punching needle 500 according to one embodiment of the present invention, it is possible to significantly reduce the risk that the follicle of the extraction target hair or the surrounding follicles are cut or damaged by the sharp cutting edge of the punching needle in a process of performing the punching operation.

Meanwhile, the punching needle 500 illustrated in FIGS. 8 and 9 is formed such that an inclined surface is formed on an inner surface of the front end portion of the punching needle to form the first cutting edge 522a having a sharp tip. However, the first cutting edge 522a may be formed such that an inclined surface is formed on an outer surface of the front end portion of the punching needle to form a cutting edge having a sharp tip or may be formed such that an inclined surface is formed on each of the inner surface and the outer surface of the front end portion of the punching needle to form a cutting edge having a sharp tip. Alternatively, in addition to the above-described shapes, the punching needle 500 according to one embodiment of the present invention may be formed in various manners such that a first cutting edge and a second cutting edge which have different shapes are formed.

For example, referring to FIGS. 10 to 13, punching needles 500 according to other embodiments of the present invention are exemplarily illustrated. The punching needle 500 of the embodiment illustrated in FIGS. 10 to 13 is formed in a shape that is generally similar to that of the above-described punching needle illustrated in FIGS. 8 and 9 and has a difference with the above-described punching needle only in the structure of the first punching portion 520a having the first cutting edge 522a of a sharp tip shape.

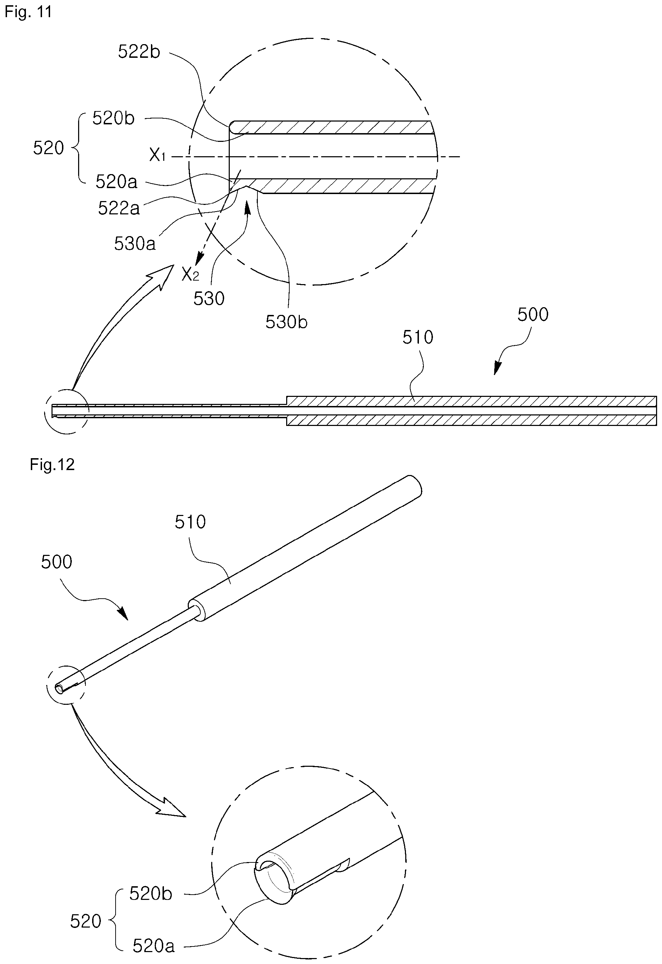

First, the punching needle 500 illustrated in FIGS. 10 and 11 is configured such that a wedge portion 530, of which diameter is reduced, is formed on an outer circumference of the first punching portion 520a to form the first cutting edge 522a having a sharp tip through the wedge portion 530. According to one embodiment of the present invention, the wedge portion 530 may be formed of a recessed groove having a reduced diameter, e.g., a groove of a triangular shape having two inclined surfaces (a front inclined surface 530a and a rear inclined surface 530b).

As shown in the drawings, since the punching needle 500 illustrated in FIGS. 10 and 11 is also formed to comprise a plurality of punching portions having different-shaped cutting edges at the front end portion of the punching needle 500, (i.e., the first punching portion 520a having the first cutting edge 522a of a sharp tip shape is provided at one side of the front end portion of the punching needle 500, and the second punching portion 520b having the second punching portion 520b of a blunt-shape is provided at the other side of the front end portion thereof), it is possible to perform a smooth punching operation while reducing the risk of damage to the follicle due to the sharp cutting edge of the punching needle, as in the above-described embodiment.

According to one embodiment of the present invention, the wedge portion 530 provided at the front end portion of the punching needle 500 may be configured such that the first cutting edge 520a, which is formed at the front end portion of the punching needle 500, is formed in a direction X₂ which is obliquely inclined with respect to the central axis X_{1.} According to the above configuration, unlike a conventional cylindrical punching needle, the punching needle 500 according to one embodiment of the present invention is configured such that the cutting edge of the front end portion, which penetrates the patient's skin, is formed outward in a radial direction instead of a direction parallel to the central axis. Consequently, when the punching needle 500 is inserted into the patient's skin, it is possible to further reduce the risk of damage to the follicle of the extraction target hair due to the punching needle.

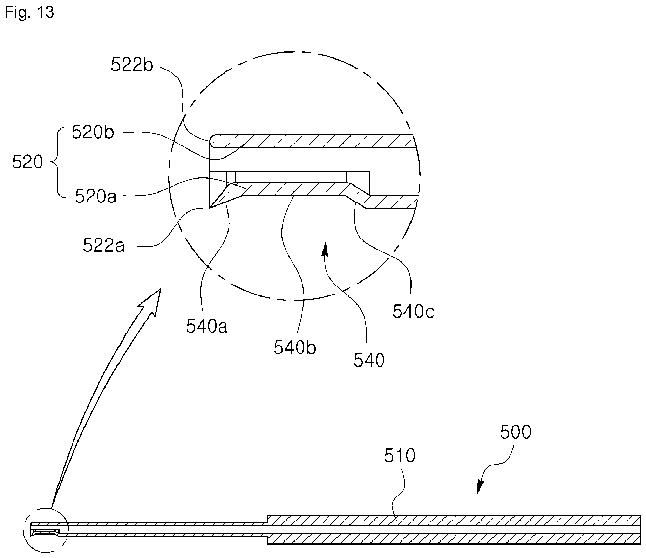

Next, the punching needle 500 illustrated in FIGS. 12 and 13 is configured to form the first punching portion 520a having the first cutting edge 522a of a sharp tip shape by forming the first punching portion as a dumbbell-shaped portion 540 (of which a cross-sectional structure is formed in a dumbbell shape), wherein the dumbbell-shaped portion 540 is constituted of a diameter reduction portion 540a of which an outer diameter and an inner diameter become narrower toward a rear side, a diameter expansion portion 540c which is located at the rear side of the diameter reduction portion 540a and of which an outer diameter and an inner diameter become larger toward a rear side, and a connection portion 540b which is located between the diameter reduction portion 540a and the diameter expansion portion 540c and formed in a cylindrical tube shape. As a result, the first cutting edge 522a having a sharp tip is formed at the front portion of the first punching portion 520a of the punching needle 500.

As shown in FIG. 13, since the punching needle 500 of the present embodiment is configured such that a sharp tip cutting edge (the first cutting edge 522a) is formed on only a portion of the front end portion, it is possible to perform a smooth punching operation while reducing the risk of damage to the follicle due to the sharp cutting edge of the punching needle, as in the above-described embodiment. Further, like the punching needle of the above-described embodiment shown in FIGS. 10 and 11, since the first cutting edge 522a of penetrating the patient's skin is formed in a direction that is inclined outward with respect to the central axis, it is possible to further reduce the risk of damage to the follicle of the extraction target hair by the punching needle when the punching needle 500 is inserted into the patient's skin. Further, the diameter reduction portion 540a formed at the front end portion is formed in a shape of which a diameter becomes narrower toward the rear side, and thus the follicle of the extraction target hair may be inserted into the punching needle 500 while being guided through an inner surface of the diameter reduction portion 540a. Consequently, as compared with the conventional punching needle having a cylindrical shape, it is possible to reduce the risk of damage to the follicle.

Furthermore, since the punching needle 500 of the embodiment shown in FIGS. 12 and 13 is configured such that the cylindrical tube-shaped connection portion 540b and the diameter expansion portion 540c of which the inner diameter and the outer diameter are increased toward the rear side are formed at the rear side of the diameter reduction portion 540a, thereby forming a structure of which the diameter is decreased and then increased at the front portion of the punching needle 500, the follicle of the hair which is extracted by the punching needle 500 may be inserted into the punching needle 500 while receiving a pulling force in a direction toward the diameter expansion portion 540c, of which the diameter is increased, passing through the connection portion 540b having the small diameter. Consequently, the risk that the follicle of the extraction target hair is cut or damaged due to the punching needle 500 can be reduced and the follicle of the extraction target hair can be extracted more stably.

Meanwhile, in the punching needle 500 according to one embodiment of the present invention, the cutting edge may be formed in a shape different from that of the above-described embodiment in various manners as long as a plurality of punching portions having different-shaped cutting edges are formed. (For example, different-shaped cutting edges may be formed by forming the first cutting edge in a sharp tip shape in which an end portion of the punching needle extends radially outward as the punching needle shown in FIG. 3, and forming the second cutting edge in a rounded blunt shape as in the above-described embodiment.) Further, in addition to the first punching portion and the second punching portion, an additional punching portion may be further provided. (For example, a third punching portion and/or a fourth punching portion, which has a cutting edge formed in a shape that is different from those of the first punching portion and the second punching portion, may be further provided.)

Further, in the above-described embodiments, although the first cutting edge 522a and the second cutting edge 522b have been formed in half at the front portion of the punching needle 500, a rate of which the first cutting edge 522a and the second cutting edge 522b are formed may be properly adjusted by a person skilled in the art. For example, the sharp first cutting edge 522a may be formed in a wide area rather than the second cutting edge 522b which is less sharp or the second cutting edge 522b which is less sharp may be formed in a wide area rather than the sharp first cutting edge 522a.

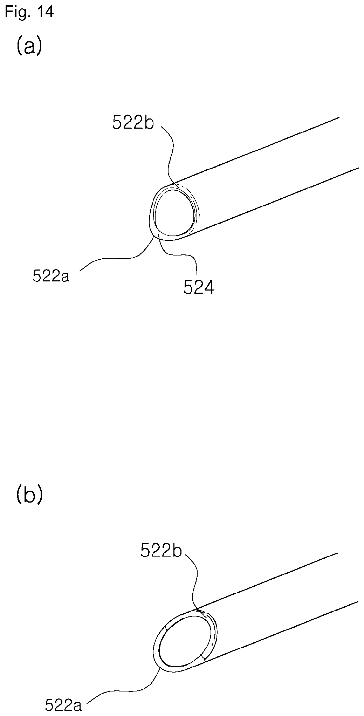

Meanwhile, according to one embodiment of the present invention, the first punching portion 520a having the first cutting edge 522a of a sharper shape may be formed to protrude forward than the second punching portion 520b having the second punching portion 520b of a less sharp shape. For example, as shown in FIG. 14 (a), the punching needle 500 according to the one embodiment of the present invention may be formed in a structure in which a portion or an entirety of the first punching portion 520a having the first cutting edge of a sharp shape is formed to protrude forward than the second punching portion 520b. Alternatively, as shown in FIG. 14 (b), the punching needle 500 according to the one embodiment of the present invention may be formed in a structure in which the front end portion of the punching needle is inclined rearward from the first punching portion 520a to the second punching portion 520b so that the first punching portion 520a protrudes forward than the second punching portion 520b. (However, the structure in which the first punching portion 520a protrudes forward than the second punching portion 520b is not limited thereto, and the present invention may be implemented in various shapes.) When the punching needle is formed such that the first punching portion 520a having a sharper cutting edge protrudes forward than the second punching portion 520b as described above, the first punching portion 520a having the sharper cutting edge may easily come into contact with the patient's skin to perform the punching operation than second punching portion 520b. Therefore, it may be ensured that the punching operation is performed more smoothly through the sharp cutting edge.

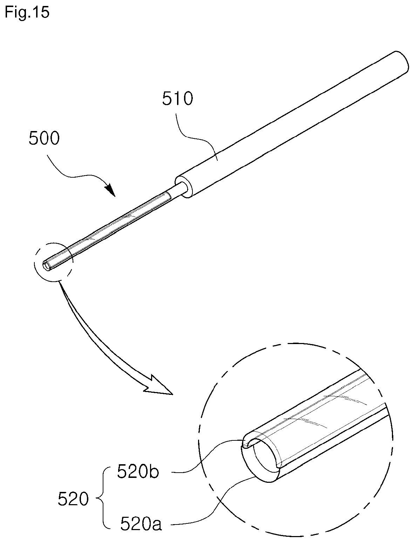

Meanwhile, as shown in FIG. 15, the punching needle 500 according to one embodiment of the present invention may be formed such that a portion or an entirety of the second punching portion 520b, in which a less sharp-shaped cutting edge is formed, is formed of a transparent material. Typically, the punching needle is generally formed of a metal material having high rigidity (e.g., stainless steel) so as to be stably penetrate into the patient's skin. However, since the second punching portion 520b of the punching needle 500 according to one embodiment of the present invention is involved in the punching operation in a state in which the patient's skin is punched to some extent by the sharp first cutting edge 522a of the first punching portion 520a, relatively high rigidity may not be required for the second punching portion 520b. Consequently, even when the second punching portion 520b is not formed of a metal material having very high rigidity, the punching operation may be sufficiently performed. According to one embodiment of the present invention, the second punching portion 520b may be formed of a material having predetermined rigidity with which the punching operation may be performed while appropriate transparency is provided, e.g., transparent acrylic, polycarbonate, polypropylene which is widely used as a material for a nonabsorbent seal or a syringe, or the like. (However, the material of the second punching portion 520b is not necessarily limited to the above-described materials, and the second punching portion 520b may be formed of any material as long as it can provide appropriate transparency and the predetermined rigidity required for the punching operation.) The second punching portion 520b may be configured to be coupled to another portion of the punching needle through various known coupling methods such as a press-fitting, welding, and the like. As described above, when the second punching portion 520b of the punching needle is formed of a transparent material, the operator can perform the punching operation while visually checking an interior of the punching needle through the second punching portion 520b. Therefore, the operator may easily insert the punching needle into the patient's skin according to a direction of hair in a state that the hair is located at a center of the punching needle. Consequently, in a process of extracting the hair, it is possible to significantly reduce a probability that the follicle is cut or damaged by the punching needle.

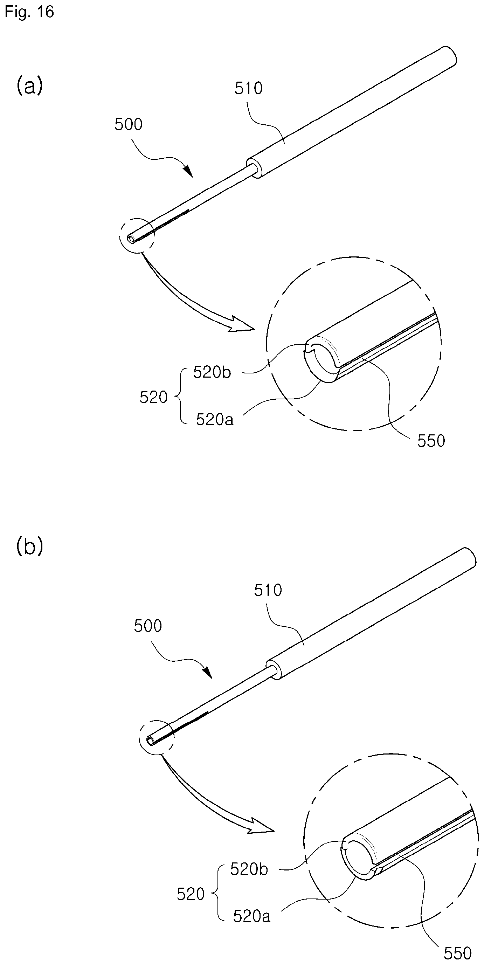

According to one embodiment of the present invention, the punching needle 500 may further comprise a cutout portion 550 extending rearward in the length direction of the punching needle 500 at one side of the front end portion. For example, referring to FIG. 16 (a), the punching needle illustrated in FIGS. 8 and 9, in which the cutout portion is additionally formed, is exemplarily illustrated. Referring to FIG. 16 (b), the punching needle illustrated in FIGS. 10 and 11, in which the cutout portion is additionally formed, is exemplarily illustrated. As such, when the cutout portion 550 is additionally formed at the front end side of the punching needle 500, the operator can perform the punching operation while visually checking the extraction target hair located inside the punching needle 500 through the cutout portion 550. That is, the operator can adjust a position and a direction of the punching needle 500 while visually directly checking the interior of the punching needle 500 through the cutout portion 550. As a result, the operator can insert the punching needle 500 into the patient's skin according to a direction of the extraction target hair in a state that the extraction target hair is easily positioned at the center of the punching needle 500, and thus it is possible to further significantly reduce the risk that the follicle is cut or damaged by the punching needle 500 in a process of extracting the extraction target hair. Further, the operator may insert the hair into the punching needle 500 from a side of the punching needle 500. Consequently, it is possible to quickly insert the hair into the punching needle 500 without cropping hairs in the extraction site and the punching operation can be performed even when hair is very long.

Meanwhile, as described above, the handpiece 200 according to one embodiment of the present invention may be formed to alternately rotate the punching needle 500 in both directions through a bidirectional electric motor or the like. As such, when the punching needle 500 is alternately rotated in the both directions, it can obtain advantages in that stress is prevented from being continuously applied to the patient's skin in the same direction to reduce damage to the patient's skin, and more stable punching process can be performed by preventing the second cutting edge 522b from coming into contact with the patient's skin at the beginning of the punching operation.

Further, according to one embodiment of the present invention, the handpiece 200 may be controlled to rotate the punching needle 500 in the both directions within an angle range in which the first cutting edge 522a of the first punching portion 520a is formed so as to prevent the second cutting edge 522b of the second punching portion 520b to come into contact with the patient's skin at the beginning of the punching operation. For example, in the case of the embodiments shown in FIGS. 8 to 13, since the first punching portion 520a and the second punching portion 520b are each formed in an angle range of about 180°, the punching needle 500 may be controlled to be rotated within the angle range of 150° in the both directions by being rotated by as much as 75° in left and right. If the operation of the handpiece 200 is controlled as described above, only the first cutting edge 522a having a sharp tip comes into contact with the patient's skin to perform the punching operation when the punching is started at the beginning of the punching operation, and thus the punching operation may be smoothly performed. However, even when the punching needle 500 is rotated at a punching angle exceeding the angle range in which the first cutting edge is formed, since the second cutting edge 522b having a less sharp shape is inserted into the skin that is incised in advance by the sharp first cutting edge 522a, the patient's skin may be punched without problems. Accordingly, it is not necessary that the punching angle of the punching needle 500 should be set within the angle range in which the first cutting edge 522a is formed.

Meanwhile, the punching angle for rotating the punching needle 500 in the both directions may be set to one fixed angle or set through a plurality of steps. In order to separate the follicle of the extraction target hair from the surrounding tissues for hair extraction, the punching needle 500 should be inserted from the epithelial layer located on an outermost side of the patient's skin to a subcutaneous fat layer located inside the dermal layer via the dermal layer located inside the epithelial layer. However, the epithelial layer and dermal layer, which are located on an outer side, are formed of relatively hard tissues, whereas the fat layer located on an inner side is comprised of very soft tissues. Therefore, when the punching needle 500 penetrates into the epithelial layer and/or the dermal layer, it is preferable to rotate the punching needle 500 at a large angle so as to effectively cut the tissues. However, when the punching needle 500 penetrates into the fat layer, the punching needle 500 may be easily inserted into the patient's skin even though the punching needle 500 is rotated at a small angle. Rather, in the case of the fat layer comprised of soft tissues, rotating the punching needle 500 at a very small angle in the both directions to operate as applying vibration may help to stably insert the punching needle 500 while reducing the risk of damage to the surrounding tissues.

For this reason, the handpiece 200 according to one embodiment of the present invention may be configured such that the punching needle 500 is rotated the at a large punching angle in an initial operation of penetrating the epithelial layer and the dermal layer at the outer side, and then the punching needle 500 is rotated at a smaller punching angle. For example, the handpiece 200 according to one embodiment of the present invention may be configured to rotate the punching needle 500 at a large punching angle by as much as a predetermined time or a predetermined number of times and then rotate the punching needle 500 at a small punching angle. According to the above-described configuration, when the punching needle 500 is inserted into the relatively hard epithelial layer and/or dermal layer, the punching needle 500 may be rotated at a large angle to be inserted thereinto, whereas when the punching needle 500 is inserted into the relatively soft fat layer, the punching needle 500 may be rotated at a small angle as being vibrated. Consequently, the punching needle 500 may be stably inserted into the patient's skin with less damage to the patient's skin. In this case, it may be more preferable that the time point for changing the punching angle, and the punching angle in each step may be adjusted and set by the operator according to a procedure situation. Further, the punching angle may be set not only in the above-described two steps but also in three or more steps.

Meanwhile, in order for the punching needle 500 according to one embodiment of the present invention to function effectively, the handpiece 200 needs to be controlled such that the punching needle 500 is always positioned in the same direction before and after the punching operation. Since a conventional electric motor used for a medical handpiece or an electric drill is controlled to start rotation when an operation signal is supplied and to stop rotation when an operation signal is blocked, if the punching needle according to one embodiment of the present invention is driven using such a conventional electric motor, when the operator takes his foot off the pedal while the electric motor is driven by operation of the pedal (manipulator), the operation signal supplied to the electric motor is blocked to stop the electric motor and the punching needle connected to the electric motor. As a result, the punching needle 500 stops in an arbitrary direction according to the time point at which the foot is released from the pedal, and thus the first cutting edge 522a and the second cutting edge 522b formed on the punching needle 500 are located at arbitrary positions.

However, in order for the punching needle 500 according to one embodiment of the present invention to function effectively, it is preferable that the punching operation begins in a state in which the first punching portion 520a having the first cutting edge 522a of a sharp tip shape is located at a position in contact with the patient's skin and the second punching portion 520b having the blunt second cutting edge 522b is located at a position opposite the position of the first punching portion 520a. More specifically, it is preferable that the punching operation begins in a state in which the punching needle 500 is located such that a central portion of the first punching portion 520a is located at a position in contact with the patient's skin, and a central portion of the second punching portion 520b is located at a position opposite the position of the first punching portion 520a. The punching operation need to begin in the above-described state such that the first punching portion 520a having the first cutting edge 522a of a sharp tip shape starts to punch and then the second punching portion 520b having the second cutting edge 522b which is less sharp is involved in the punching process after the patient's skin starts to be penetrated.

To this end, the handpiece 200 according to one embodiment of the present invention may be configured to terminate the punching operation in a state that the punching needle 500 returns to its original position before the punching operation (i.e., rotation of the electric motor and the punching needle 500 is terminated in a state that the punching needle 500 returns to its original position before the punching operation).

Such a position return function of the punching needle 500 may be implemented in the following manner. For example, in the handpiece 200 according to one embodiment of the present invention, an operation, in which the punching needle 500 completes a rotation pattern that the punching needle 500 is alternately rotated in the both directions and then returns to its original position, may be regarded as one set of the punching operation. When the operation signal provided from the power generator 220 is blocked while the handpiece 200 is driven, the power generator 220 is controlled to be further driven so as to allow the punching needle 500 to be rotated until one set of the ongoing punching operation is completed and then to be stopped such that the position return function may be implemented.

For example, when the operator operates the manipulator 400, the handpiece 200 according to one embodiment of the present invention alternately rotates the punching needle 500 in the both directions using a driving force which is generated by the power generator 220, and thus the punching needle 500 may be stably inserted into the patient's skin. Thereafter, when the operator stops the operation of the manipulator 400 so as to terminate the punching operation, the operation signal supplied to the power generator 220 is blocked. In this case, unlike the conventional electric motor, the handpiece 200 according to one embodiment of the present invention may be configured to control to stop the punching needle 500 after one set of the ongoing punching operation performed by the punching needle 500 is completed, instead of immediately stopping the power generator 220 (electric motor) at an instant when the operation of the manipulator 400 is stopped.

As described above, in accordance with the configuration for returning the punching needle 500 to its original position, the punching needle 500 may always be located in the same position before and after the punching operation. Thus, the first punching portion 520a and the second punching portion 520b may be automatically located at a position required for the punching operation without the operator having to turn and hold the handpiece 200 whenever the operator performs the punching operation, and thus the rapid and accurate punching operation can be performed.

While the present invention has been described with reference to the specific matters such as the specific components, the limited embodiments and the drawings, the above description is provided for better understanding of the present invention, this present invention is not limited to the above-described embodiment, and those skilled in the art to which the present invention pertains will make that various changes and modifications from the above disclosure.

Accordingly, the spirit of the present invention should not be limited to the above-described embodiments, and the appended claims as well as the equivalents and the modifications thereof should be construed as being fall within the spirit and scope of the present invention.

## Claims

1. A punching needle 500 for a handpiece used to extract hair from patient's skin, wherein:
an accommodation space, into which follicle of extraction target hair may be inserted, is formed in the inside of a front portion of the punching needle 500;
a punching portion 520 of contacting with the patient's skin and punching the patient's skin is provided in the front end portion of the punching needle 500;
the punching portion 520 comprises a first punching portion 520a and a second punching portion 520b which have different-shaped cutting edges, and
a first cutting edge 522a provided in the first punching portion 520a is formed in a shape that is sharper than that of a second cutting edge 522b provided in the second punching portion 520b.

2. The punching needle of claim 1, wherein the first punching portion 520a is formed to decrease a thickness of the punching needle toward a front side such that the first cutting edge 522a having a sharp tip is formed in a front end portion.

3. The punching needle of claim 1, wherein:
the first punching portion 520a has a wedge portion 530 of a recessed shape on an outer circumference of a front portion such that the first cutting edge 522a having the sharp tip is formed at the front portion of the first punching portion 520a due to the wedge portion 530; and
the first cutting edge 522a formed at the front portion of the first punching portion 520a is formed in a direction X2 that is obliquely inclined with respect to a central axis X1 of the punching needle due to the wedge portion 530.

4. The punching needle of claim 3, wherein the wedge portion 530 formed on the outer circumference of the front portion of the first punching portion 520a is formed of a recess of a triangular shape having a front inclined surface 530a and a rear inclined surface 530b.

5. The punching needle of claim 1, wherein:
the first punching portion 520a comprises a dumbbell-shaped portion 540 having a diameter reduction portion 540a which is located at the front end portion and of which an outer diameter and an inner diameter become smaller toward a rear side; a diameter expansion portion 540c which is located at the rear side of the diameter reduction portion 540a and of which an outer diameter and an inner diameter becomes larger toward a rear side; and a connection portion 540b which is formed in a cylindrical tube shape and which connects between the diameter reduction portion 540a and the diameter expansion portion 540c; and
the first cutting edge 522a having a sharp tip is formed at the front end portion of the diameter reduction portion 540a.

6. The punching needle of any one of claims 1 to 5, wherein an end portion of the second cutting edge 522b provided in the second punching portion (520b) is rounded so that the portion, which comes into contact with the patient's skin, is formed in a blunt shape.

7. The punching needle of claim 6, wherein the first punching portion 520a is formed such that an entirety or a portion of the front end portion thereof protrudes forward than the front end portion of the second punching portion 520b.

8. The punching needle of claim 6, wherein an entirety or a portion of the second punching portion 520b is formed of a transparent material.

9. The punching needle of claim 6, further comprising:
a cutout portion 550 formed to extend rearward in a length direction of the punching needle at one side of the front portion of the punching needle 500.

10. A handpiece 200 used to extract hair from patient's skin, comprising:
a housing 210;
a power generator 220 provided in the housing 210;
a power transmitting part 230 configured to transmit a rotating force generated from the power generator 220; and
a punching needle 500 engaged with a front portion of the power transmitting part 230 and inserted into the patient's skin while being rotated due to the rotating force generated from the power generator 220,
wherein an accommodation space, into which follicle of extraction target hair may be inserted, is formed in the inside of a front portion of the punching needle 500,
a punching portion 520 of contacting with the patient's skin and punching the patient's skin is provided in the front end portion of the punching needle 500,
the punching portion 520 comprises a first punching portion 520a and a second punching portion 520b which have different-shaped cutting edges, and
a first cutting edge 522a provided in the first punching portion 520a is formed in a shape that is sharper than that of a second cutting edge 522b provided in the second punching portion 520b.

11. The handpiece of claim 10, wherein the first punching portion 520a is formed to decrease a thickness of the punching needle toward a front side such that the first cutting edge 522a having a sharp tip is formed at a front end portion.

12. The handpiece of claim 10, wherein:
the first punching portion 520a has a wedge portion 530 of a recessed shape on an outer circumference of a front portion such that the first cutting edge 522a having the sharp tip is formed at the front portion of the first punching portion 520a due to the wedge portion 530; and
the first cutting edge 522a formed at the front portion of the first punching portion 520a is formed in a direction X2 that is obliquely inclined with respect to a central axis X1 of the punching needle due to the wedge portion 530.

13. The handpiece of claim 12, wherein the wedge portion 530 formed on the outer circumference of the front portion of the first punching portion 520a is formed of a recess of a triangular shape having a front inclined surface 530a and a rear inclined surface 530b.

14. The handpiece of claim 10, wherein:
the first punching portion 520a comprises a dumbbell-shaped portion 540 having a diameter reduction portion 540a which is located at the front end portion and of which an outer diameter and an inner diameter become smaller toward a rear side; a diameter expansion portion 540c which is located at the rear side of the diameter reduction portion 540a and of which an outer diameter and an inner diameter becomes larger toward a rear side; and a connection portion 540b which is formed in a cylindrical tube shape and which connects between the diameter reduction portion 540a and the diameter expansion portion 540c; and
the first cutting edge 522a having a sharp tip is formed at the front end portion of the diameter reduction portion 540a.

15. The handpiece of any one of claims 10 to 14, wherein an end portion of the second cutting edge 522b provided in the second punching portion (520b) is rounded so that the portion, which comes into contact with the patient's skin, is formed in a blunt shape

16. The handpiece of claim 15, wherein the first punching portion 520a is formed such that an entirety or a portion of the front end portion thereof protrudes forward than the front end portion of the second punching portion 520b.

17. The handpiece of claim 15, wherein an entirety or a portion of the second punching portion 520b is formed of a transparent material.

18. The handpiece of claim 15, wherein the punching needle further comprises a cutout portion 550 formed to extend rearward in a length direction of the punching needle at one side of the front portion of the punching needle 500.

19. The handpiece of claim 10, wherein the power generator 220 is driven to alternately rotate the punching needle 500 in both directions.

20. The handpiece of claim 19, wherein the power generator 220 is driven to rotate the punching needle 500 in the both directions within an angle range in which the first punching portion 520a is formed.

21. The handpiece of claim 10, wherein the power generator 220 is controlled to start rotating the punching needle in response to a manipulation command of an operator through a manipulator 400 and, when the manipulation command of the operator through the manipulator 400 is released, the power generator 220 is controlled to terminate the rotation of the punching needle in a state of returning the punching needle 500 to the position before the punching needle 500 is operated.
